# EUROPEAN PATENT SPECIFICATION

(11) **EP 1 871 790 B1**
(45) Date of publication and mention of the grant of the patent: **20.03.2013**
(21) Application number: 06833668.4
(22) Date of filing: 21.11.2006
(51) Int. Cl.: C07K 14/395, C12N 9/02, C12G 3/02, C12N 15/81, C12Q 1/00, C12Q 1/68, C12C 12/00

(54) **GENE ENCODING PROTEIN WITH VICINAL DIKETONE OR DIACETYL-REDUCING ACTIVITY AND USE THEREOF**
GEN CODIEREND EIN PROTEIN MIT VICINALDIKETON- ODER DIACETYLREDUZIERENDER AKTIVITÄT UND SEINE VERWENDUNG
GÈNE CODANT POUR UNE PROTÉINE À ACTIVITÉ RÉDUCTRICE DE DICÉTONES VICINALES OU DE DIACÉTYLE ET UTILISATION DUDIT GÈNE

(30) Priority: 24.02.2006 JP 2006048957
(43) Date of publication of application: 02.01.2008
(73) Proprietor: Suntory Holdings Limited, Kita-ku, Osaka-shi, Osaka 530-8203 (JP)
(72) Inventor: NAKAO, Yoshihiro, c/o SUNTORY LIMITED, Mishima-gun, Osaka 618-8503 (JP); KODAMA, Yukiko, Mishima-gun, Osaka 618-8503 (JP); SHIMONAGA, Tomoko c/o SUNTORY LIMITED, Mishima-gun Osaka 6188503 (JP); OMURA, Fumihiko, Mishima-gun, Osaka 618-8503 (JP)
(74) Representative: Vossius & Partner
(86) International application number: PCT/JP2006/323865
(87) International publication number: WO 2007/097088

(56) References cited:
- EP-A1- 1 568 763
- US-A1- 2004 265 862
- KIM JONG-MYONG ET AL: "A member of the YER057c/Yjgf/Uk114 family links isoleucine biosynthesis and intact mitochondria maintenance in Saccharomyces cerevisiae" GENES TO CELLS, vol. 6, no. 6, June 2001 (2001-06), pages 507-517, XP002419960 ISSN: 1356-9597
- BAMFORTH C W ET AL: "ENZYMOLOGY OF VICINAL DIKETONE REDUCTION IN BREWER'S YEAST" JOURNAL OF THE INSTITUTE OF BREWING, INSTITUTE OF BREWING. LONDON, GB, vol. 110, no. 2, 2004, pages 83-93, XP001248222 ISSN: 0046-9750
- VILLANUEBA K D ET AL: "SUBTHRESHOLD VICINAL DIKETONES LEVELS IN LAGER BREWING YEAST FERMENTATIONS BY MEANS OF ILV5 GENE AMPLIFICATIONS" JOURNAL OF THE AMERICAN SOCIETY OF BREWING CHEMISTS, AMERICAN SOCIETY OF BREWING CHEMISTS, ST PAUL, MN, US, vol. 48, no. 3, 1990, pages 111-114, XP008070978 ISSN: 0361-0470
- LIU Z ET AL: "Constructing an amylolytic brewing yeast Saccharomyces pastorianus suitable for accelerated brewing" JOURNAL OF BIOSCIENCE AND BIOENGINEERING, ELSEVIER, AMSTERDAM,, NL, vol. 98, no. 6, 2004, pages 414-419, XP004727084 ISSN: 1389-1723
- OKABE MITSUYASU ET AL: "Continuous beer fermentation by high cell-density culture of bottom brewer's yeast" JOURNAL OF FERMENTATION AND BIOENGINEERING, vol. 77, no. 1, 1994, pages 41-45, XP002419961 ISSN: 0922-338X

## Description

### TECHNICAL FIELD

The present invention relates to a gene encoding a protein having a vicinal diketone or diacetyl-reducing activity and use thereof, in particular, a brewery yeast for producing alcoholic beverages with superior flavor, and a method for producing said beverages. More particularly, the present invention relates to a yeast, whose capability of producing vicinal diketone(s), especially diacetyl, that are responsible for off-flavors in products, is reduced by amplifying expression level of MMF1 gene encoding a protein Mmflp) having a vicinal diketone or diacetyl-reducing activity, especially non-ScMMF1 gene or ScMMF1 gene specific to a lager brewing yeast, and to a method for producing alcoholic beverages with said yeast

### BACKGROUND ART

Flavor of Diacetyl, hereinafter also referred to as "DA", is a representative off-flavor in brewed alcoholic beverages such as beer, sake and wine and so on among flavoring substances of alcoholic beverages. DA flavor, which is also referred to as "butter flavor" or "sweaty flavor" in beer, "tsuwari-ka", which means a nauseating flavor, in sake, occurs when vicinal dketone(s), hereinafter also referred to as "VDK", mainly DA, are present above certain threshold levels in products. The threshold level is said to be 0.1 ppm (parts per million) in beer (Journal of the Institute of Brewing, 76, 486 (1979)).

VDK in alcoholic beverages can be broadly divided into DA and 2,3-pentanedione, herein after referred to as "PD". DA and PD are formed by non-enzymatic reactions, which yeasts are not involved in, of α-acetolactic-acid and α-acetohydroxybutyric-acid as precursors which are intermediate in biosynthesis of valine and isoleucine, respectively.

According to these information, VDKs (i.e., DA and PD) and their precursors α-acetohydroxy-acids (i.e., α-acetolactic-acid and α-acetohydroxybutyric-acid) are thought to be the compounds which can impart DA flavors to products. Accordingly, breeding of yeasts which steadily reduces these compounds makes manufacturing control of alcoholic beverages easy as well as expands capability of developing new products:

A method for suppressing production of DA by using rice-malt-yeast culture containing low level of pyruvic acid which is a precursor of acetohydroxy-acids in production of sake in, for example, Japanese Patent Application Laid-Open No. 2001-204437. It is also reported that production of VDKs are reduced in valine, leucine and isoleucine auxotrophic yeast in beer production. However, the yeast has not come into practical use since auxotrophic strains tend to show retarded growth/fermentation. Japanese Patent Application Laid-Open NO. 2002-291465 discloses a method obtaining variant strains sensitive to analogues of the branched amino acids described above, and selecting DA low accumulating strains from the variant strains. Genetically engineered yeasts derived from laboratory-designed yeasts whose amount of expression of ILV5 gene is regulated is reported in Journal of American Society of Brewing Chemists, Proceeding, 81-84 (1987), and also genetically engineered yeast whose amount of expression of ILV3 gene is regulated is reported in European Brewery Convention, Proceedings, of the 21st EBC congress, Madrid, 553-560 (1987). The enzymatic activity of the acetohydroxy-acid reductoisomerase encoded by ILV5 gene is increased 5 to 7-fold, and the amount of production of VDKs are reduced to about 40% in the case.

Villanueba et al. (American Society of Brewing Chemists Journal 48 (1990), 111-114) report on the reduction of vicinal diketone (VDK) levels in fermentation by amplification of the ILV5 gene.

Besides, the enzymatic activity of the dihydroxy-acid dehydratase encoded by ILV3 is increased 5 to 6-fold. On the contrary, no significant reduction of the amount of production of VDKs was observed. However, any influence on practical beer brewing is analyzed in the two reports described above where synthetic media are used. On the other hand, Villa et al. reported in Journal of American Society of Brewing Chemists, 53;49-53 (1995), that plasmid amplification of the gene products of ILV5, ILV3 or under ILV5+ILV3 in brewer's yeast resulted in VDK. decreases of 70, 40 and 60% respectively, when compared to that of normal brewer's yeast on practical beer brewing.

Also, Dulieu et al. proposed a method converting α-acetolactic-acid, which served as a precursor of DA, rapidly to acetoin using α-acetolactate decarboxylase in European Brewery Convention, Proceedings of the 26th EBC congress, Maastricht, 455-460 (1997). However, α-acetolactate decarboxylase is an enzyme prepared only by utilizing recombinant DNA technology, and thus use of the enzyme is not acceptable due to consumers' negative images in Japan. Genetically engineered yeasts using DNA strands encoding α-acetolactate decarboxylase are reported in both Japanese Patent Application Laid-Open Nos. H2-26545 and H07-171.

Bamforth and Kanauchi (J. Inst. Brew. 110 (2004), 83-93) describes the enzymology of vicinal diketone reduction in brewer's yeast and describes that ale and lager strains contain multiple enzymes capable of reducing diacetyl and pentanedione. Liu et al. (J. Biosci. Bioengineering 98 (2004), 414-419) describe a genetically modified yeast of the species Saccharomyces pastorianus in which the α-acetolactate synthase gene is disrupted and an d-amylase gene is introduced as a selective marker. Its α-acetolactate synthase activity was lowered by 30% and during fermentation the diacetyl concentration was reduced by 70%.

Okabe et al. (J. Fermentation and Bioengineering 77 (1994), 41-45) disclose a continuous beer fermentation method by high cell-density culture of bottom brewer's yeast which leads to a reduction of vicinal diketone content of the beer.

### DISCLOSURE OF INVENTION

Under the circumstances described above, there were demands for developing a method for producing alcoholic beverages with superior flavor by breeding a yeast with low VDK-producing ability utilizing a gene encoding a protein capable of reducing the smell of VDKs (vicinal diketones), especially DA (diacetyl), and the protein.

To solve the problems described above, the present inventors made extensive studies, and as a result succeeded in identifying and isolating a gene encoding a protein having a vicinal diketone or diacetyl-reducing activity. Moreover, a yeast in which the obtained gene was transformed and expressed was produced to confirm reduction of the VDK concentration, especially DA concentration, thereby completing the present invention.

Thus, the present invention relates to a gene encoding a protein having a vicinal diketone or diacetyl-reducing activity (activity of reducing a vicinal diketone(s) or diacetyl, existing specifically in a lager brewing yeast to a protein encoded by said gene, to a transformed yeast in which the expression of said gene is controlled, to a method for controlling the level of VDKs, especially the level of DA in a product by using a yeast in which the expression of said gene is controlled. More specifically, the present invention provides the following polynucleotides, a vector comprising said polynucleotide, a transformed yeast introduced with said vector, a method for producing alcoholic beverages by using said transformed yeat, and the like.
(1) A polynucleotide selected from the group consisting of:
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2.
   (c) a polynucleotide comprising a polynucleotide encoding a protein consisting of the amino acid sequence of SEQ ID NO:2 with one to 8 amino acids thereof being deleted, substituted, inserted and/or added, and having a vicinal diketone or diacetyl-reducing activity; and
   (d) a polynucleotide comprising a polynucleotide encoding a protein having an amino acid sequence having 94% or higher identity with the amino acid sequence of SEQ ID NO:2, and having a vicinal diketones or diacetyl-reducing activity.
(2) The polynucleotide of (1) above selected from the group consisting of:
   (g) a polynucleotide encoding a protein consisting of the amino acid sequence of SEQ ID NO: 2, or encoding an amino acid sequence of SEQ ID NO: 2 wherein 1 amino acid thereof is deleted, substituted, inserted, and/or added, and wherein said protein has a vicinal diketone or diacetyl-reducing activity; and
   (h) a polynucleotide encoding a protein having 99% or higher identity with the amino acid sequence of SEQ ID NO: 2, and having a vicinal diketone or diacetyl-reducing activity.
(3) The polynucleotide of (1) above comprising a polynucleotide consisting of SEQ ID NO: 1.
(4) The polynucleotide of (1) above comprising a polynucleotide encoding a protein consisting of SEQ ID NO: 2.
(5) The polynucleotide of any one of (1) to (4) above, wherein the polynucleotide is DNA.
(6) A protein encoded by the polynucleotide of any one of (1) to (5) above.
(7) A vector comprising the polynucleotide of any one of (1) to (5) above.
(8a) Also disclosed is a vector of (7) above, which comprises an expression cassette comprising the following components:
   (x) a promoter that can be transcribed in a yeast cell:
   (y) any of the polynucleotides described in (1) to (5) above linked to the promoter in a sense or antisense direction; and
   (z) a signal that can function in a yeast with respect to transcription termination and polyadenylation of a RNA molecule.
(9) A yeast comprising the vector of (7).
(10) The yeast of (9) above, wherein a total vicinal diketone or total diacetyl-producing capability is reduced by introducing the vector of (7) above.
(11) The yeast of (10) above, wherein a total vicinal diketone or total diacetyl-producing capability is reduced by increasing an expression level of the protein of (6) above.
(12) A method for producing an alcoholic beverage comprising culturing the yeast of any one of (9) to (11) above.
(13) A method for producing an alcoholic beverage with reduced amount of vicinal diketone or diacetyl, comprising culturing a yeast containing a vector comprising the polynucleotide selected from the group consisting of:
   (j) a polynucleotide encoding a protein consisting of the amino acid sequence of SEQ ID NO: 4, or encoding an amino acid sequence of SEQ ID NO: 4 wherein 1 to 10 amino acids thereof is deleted, substituted, inserted, and/or added, and wherein said protein has a vicinal diketone or diacetyl-reducing activity;
   (k) a polynucleotide encoding a protein having 90% or higher identity with the amino acid sequences of SEQ ID NO: 4, and having a vicinal diketone or diacetyl-reducing activity; and
   (I) a polynucleotide which hybridizes to SEQ ID NO: 3 or which hybridizes to a nucleotide sequence complementary to the nucleotide sequence of SEQ ID NO: 3 under high stringent conditions, and which encodes a protein having a vicinal diketone or diacetyl-reducing activity.
(14) The method for producing an alcoholic beverage of (12) or (13) above, wherein the brewed alcoholic beverage is a malt beverage.
(15) The method for producing an alcoholic beverage of (12) or (13) above, wherein the brewed alcoholic beverage is wine.
(16) A method for assessing a test yeast for its total vicinal diketone or total diacetyl-producing capability, comprising using a primer or a probe designed based on a nucleotide sequence of a gene having the nucleotide sequence of SEQ ID NO: or SEQ ID NO:3, and encoding a protein having a vicinal diketone or diacetyl-reducing ability.
(16a) Also disclosed is a method for selecting a yeast having a low total vicinal diketone or total diacetyl-producing capability by using the method described in (16) above.
(16b) Also disclosed is a method for producing an alcoholic beverage (for example, beer) by using the yeast selected with the method in (16a) above.
(17) A method for assessing a test yeast for its total vicinal diketone or total diacetyl-producing capability, comprising: culturing a test yeast; and measuring an expression level of a gene having the nucleotide sequence of SEQ ID NO:1 or SEQ ID NO:3, and encoding a protein having a vicinal diketone or diacetyl-reducing ability.
(17a) Also disclosed is a method for selecting a yeast having a low total vicinal diketone or total diacetyl-producing capability, which comprises assessing a test yeast by the method described in (17) above and selecting a yeast having a high expression level of a gene encoding a protein having a vicinal diketone or diacetyl-reducing activity.
(17b) Also disclosed is a method for producing an alcoholic beverage (for example, beer) by using the yeast selected with the method in (17a) above.
(18) A method for selecting a yeast, comprising: culturing test yeasts; quantifying the protein according to (6) or measuring an expression level of a gene having the nucleotide sequence of SEQ ID NO:1 or SEQ ID NO:3, and encoding a protein having a vicinal diketone or diacetyl-reducing ability; and selecting a test yeast having said protein amount or said gene expression level according to a target total vicinal diketone or total diacetyl-producing capability.
(18a) Also disclosed is a method for selecting a yeast, comprising: culturing test yeasts; measuring a total vicinal diketone or total diacetyl-producing capability; and selecting a test yeast having a target total vicinal diketone or total diacetyl-producing capability.
(19) The method for selecting a yeast according to (18) above, comprising: culturing a reference yeast and test yeasts; measuring an expression level of a gene having the nucleotide sequence of SEQ ID NO: 1 or SEQ ID NO: 3, and encoding a protein having a vicinal diketone or diacetyl-reducing ability in each yeast; and selecting a test yeast having the gene expressed higher than that in the reference yeast.
(20) The method for selecting a yeast according to (18) above, comprising: culturing a reference yeast and test yeasts; quantifying the protein according to (6) above in each yeast; and selecting a test yeast having said protein for a larger amount than that in the reference yeast.
(21) A method for producing an alcoholic beverage comprising: conducting fermentation for producing an alcoholic beverage using the yeast according to any one of (9) to (11) above or a yeast selected by the method according to any one of (18) to (20) above; and adjusting the total vicinal diketone or total diacetyl-producing capability.

According to the method for producing alcoholic beverages of the present invention, because of reduction of the production amount of VDKs, especially DA, which are responsible for off-flavors in products, alcoholic beverages with superior flavor can be readily produced.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract consumption with time upon beer fermentation test The horizontal axis represents fermentation time while the vertical axis represents apparent extract concentration (w/w%).
Figure 3 shows the expression behavior of non-ScMMF1 gene in yeasts upon beer fermentation test. The horizontal axis represents fermentation time while the vertical axis represents the brightness of detected signal.
Figure 4 shows the cell growth with time upon beer fermentation test using the non-ScRMMF1-highly expressed strain. The horizontal axis represents fermentation time while the vertical axis represents optical density at 660 nm (OD660).
Figure 5 shows the extract consumption with time upon beer fermentation test using the non-ScMMF1-highly expressed strain. The horizontal axis represents fermentation time while the vertical axis represents apparent extract concentration (w/w%).
Figure 6 shows the VDK concentration in the fermentation broth (at the completion of fermentation) during beer fermentation test using the non-ScMMF1-highly expressed strain.
Figure 7 shows the cell growth with time upon beer fermentation test The horizontal axis represents fermentation time while the vertical axis represents optical density at 660 nm (OD660).
Figure 8 shows the extract consumption with time upon beer fermentation test The horizontal axis represents fermentation time while the vertical axis represents apparent extract concentration (w/w/%).
Figure 9 shows the expression behavior of ScMMF1 gene in yeasts upon beer fermentation test. The horizontal axis represents fermentation time while the vertical axis represents the brightness of detected signal.
Figure 10 shows the cell growth with time upon beer fermentation test using the ScMMF1-highly expressed strain The horizontal axis represents fermentation time while the vertical axis represents optical density at 660 nm (OD660).
Figure 11 shows the extract consumption with time upon beer fermentation test using the ScMMF1-highly expressed strain. The horizontal axis represents fermentation time while the vertical axis represents apparent extract concentration (w/w%).
Figure 13 shows the VDK concentration in the fermentation broth (at the completion of fermentation) during beer fermentation test using the ScMMF1-highly expressed strain.

### BEST MODES FOR CARRYING OUT THE INVENTION

The present inventors isolated and identified non-ScMMF1 gene and ScMMF1 gene encoding a protein having a vicinal diketone or diacetyl-reducing activity unique to lager brewing yeast based on the lager brewing yeast genome information mapped according to the method disclosed in Japanese Patent Application Laid-Open No. 2004-283169. These nucleotide sequences of the genes are represented by SEQ ID NO: 1 and SEQ ID NO: 3, respectively. Further, each of an amino acid sequence of a protein encoded by each of these genes is represented by SEQ ID NO: 2 or SEQ ID NO: 4, respectively.

### 1. Polynucleotide of the invention

First of all, the present invention provides (a) a polynucleotide comprising a polynucleotide of the nucleotide sequence of SEQ ID NO:1 ; and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding a protein having a vicinal diketone or diacetyl-reducing activity derived from lager brewing yeast and may include other polynucleotides encoding proteins having equivalent functions to said protein. Proteins with equivalent functions include, (c) a protein of an amino acid sequence of SEQ ID NO: 2 with one to 8 amino acids thereof being deleted, substitute, inserted and/or added and having a vicinal diketone or diacetyl-reducing activity.

Such proteins include a protein consisting of an amino acid sequence of SEQ ID NO: 2 with 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having a vicinal diketone or diacetyl-reducine activity. In general, the number of deletions, substitutions, insertions, and/or additions is preferably smaller. In addition, such proteins include (d) a protein having an amino acid sequence with 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO:2 ; and having a vicinal diketone or diacetyl-reducing activity. In general, the percentage identity is preferably higher.

The vicinal diketone or diacetyl-reducing activity can be assessed, for example by, a method of Drews, et al. (Drews et al., Mon. fur Brau, 34, 1966) wherein total VDK (DA and PD) concentration is measured in fermentation broth and compared.

Furthermore, the present invention also relates to a method for producing an alcoholic beverage with reduced amount of vicinal diketone or diacetyl, comprising culturing a yeast containing a vector comprising the polynucleotide selected from the group consisting of:
(j) a polynucleotide encoding a protein consisting of the amino acid sequence of SEQ ID NO: 4, or encoding an amino acid sequence of SEQ ID NO: 4 wherein 1 to 10 amino acids thereof is deleted, substituted, inserted, and/or added, and wherein said protein has a vicinal diketone or diacetyl-reducing activity;
(k) a polynucleotide encoding a protein having 90% or higher identity with the amino acid sequence of SEQ ID NO: 4, and having a vicinal diketone or diacetyl-reducing activity; and
(I) a polynucleotide which hybridizes to SEQ ID NO: 3 or which hybridizes to a nucleotide sequence complementary to the nucleotide sequence of SEQ ID NO: 3 under high stringent conditions, and which encodes a protein having a vicinal diketone or diacetyl-reducing activity.

The polynucleotide used in said method of the present invention is not limited to the polynucleotide encoding a protein having a vicinal diketone or diacetyl-reducing activity derived as shown in SEQ ID NO:4 but may include other polynucleotides encoding proteins having equivalent functions to said protein. Proteins with equivalent functions include, (c) a protein of an amino acid sequence of SEQ ID NO:4 with one to 10 amino acids thereof being deleted, substituted, inserted and/or added and having a vicinal diketone or diacetyl-reducing activity.

Such proteins include a protein consisting of an amino acid sequence of SEQ ID NO:4 with 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having a vicinal diketone or diacetyl-reducing activity. In general, the number of deletions, substitutions, insertions, and/or additions is preferably smaller. In addition, such proteins include (d) a protein having an amino acid sequence with 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO:4, and having a vicinal diketone or diacetyl-reducing activity. In general, the percentage identity is preferably higher.

As pointed out above, the present invention also contemplates that the vector used in the method according to the invention comprises (1) a polynucleotide comprising a polynucleotide which hybridizes to a polynucleotide consisting of a nucleotide sequence complementary to the nucleotide sequence of SEQ ID NO:3 under stringent conditions and which encodes a protein having a vicinal diketone or diacetyl-reducing activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO:3.

The hybridization method may be a method described, for example, in Molecular Cloning 3rd Ed., Current Protocols in Molecular Biology, John Wiley & Sons 1987-1997.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution. 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Phamiacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Identity between amino acid sequences or nucleotide sequences may be determined using algorithm BLAST by Karlin and Altschul (Proc. Natl. Acad. Sci. USA, 87: 2264-2268, 1990; Proc. Natl. Acad. Sci. USA. 90: 5873, 1993). Programs called BLAST and BLAST based on BLAST algorithm have been developed (Altschul SF et al., J. Mol. Biol. 215: 403, 1990). When a nucleotide sequence is sequenced using BLASTN, the parameters are, for examples, score = 100 and word length = 12. When an amino acid sequence is sequenced using BLASTX, the parameters are, for example, score = 50 and word length = 3. When BLAST and Gapped BLAST programs are used, default parameters for each of the programs are employed.

### 2. Protein of the present invention

The present invention also provides proteins encoded by any of the polynucleotides (a), (b), (c), (d), (g) and (h) above. A preferred protein of the present invention comprises an amino acid sequence of SEQ ID NO:2 with one to 8 amino acids thereof being deleted, substituted, inserted and/or added, and has a vicinal diketone or diacetyl-reducing activity.

Such protein includes those having an amino acid sequence of SEQ ID NO: 2
with amino acid residues thereof of the number mentioned above being deleted, substituted, inserted and/or added and having a vicinal diketone or diacetyl-reducing activity. In addition, such protein includes those having homology of 94% or more, or the most preferably about 95% or more as described above with the amino acid sequence of SEQ ID NO: 2 and having a vicinal diketone or diacetyl-reducing activity.

Such proteins may be obtained by employing site-directed mutation described, for example, in Molecular Cloning 3rd Ed., Current Protocols in Molecular Biology, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of one to 8 amino acid residues in an amino acid sequence of the protein of the invention means that one to 8 amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Ground A: leucine, isoleucine, norleucine, valine, norvaline, alanine, 2-aminobutanoic acid, methionine, o-methylserine, t-butylglycine, t-butytalanine, cyclohexylalanine; Group B: asparatic acid, glutamic acid, isoasparatic acid, isoglutamic acid. 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline. 3-hydroxyproline, 4-hydroxyproline: Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega. PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides (such as DNA) described in (a) to (d), (g) or (h) above. Generally, the vector of the present invention comprises an expression cassette including as components (x) a promoter that can transcribe in a yeast cell; (y) a polynucleotide (such as DNA) described in any of (a) to (d), (g) or (h) above that is linked to the promoter in sense or antisense direction; and (z) a signal that functions in the yeast with respect to transcription termination and polyadenylation of RNA molecule.

A vector introduced in the yeast may be any of a multicopy type (YEp type), a single copy type (YCp type), or a chromosome integration type (YIp type). For example, YEp24 (J. R Broach et al., Experimental Manipulation of Gene Expression, Academic Press, New York. 83, 19S3) is known as a YEp type vector, YCp50 (M. D. Rose et al., Gene 60:237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they have no influence on the concentration of constituents such as amino acid and extract in fermentation broth: For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660,1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example; *Saccharomyces pastorianus* W34/70, *Saccharomyces carlsbergensis* NCYC453 or NCYC456, or *Saccharomyces cerevisiae* NBRC1951, NBRC1952, NBRC1953 or NBRC1954 may be used. In addition, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl. Acad. Sci. USA, 75: 1929(1978)), a lithium acetate method (J. Bacteriology, 153: 163 (1983)), and methods described in Proc. Natl. Acad, Sci. USA, 75: 1929 (1978), Methods in Yeast Genetics, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali ion metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4.000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in Molecular Cloning 3rd. Ed., and Methods in Yeast Genetics, A Laboratory Manual (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to reduce the level of VDKs, especially DA, of desired alcoholic beverages, and produce alcoholic beverages having enhanced flavor. In addition, yeasts to be selected by the yeast assessment method of the present invention can also be used. The target alcoholic beverages include, for example, but not limited to beer, sparkling liquor (*happoushu*) such as a beer-taste beverage, wine, whisky, sake and the like.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with an decreased level of VDKs, especially DA. Thus, according to the present invention, alcoholic beverages with enhanced flavor can be produced using the existing facility without increasing the cost.

### 5. Yeast assessment method of the invention

The present invention relates to a method for assessing a test yeast for its capability of producing total vicinal diketones or capability of producing total diacetyl by using a primer or a probe designed based on a nucleotide sequence of a gene having the nucleotide sequence of SEQ ID NO:1 or SEQ ID NO: 3, and encoding a protein having a vicinal diketone or diacetyl-reducing activity. General techniques for such assessment method is known and is described in, for example, WO01/040514, Japanese Laid-Open Patent Application No. 8-205900 or the like. This assessment method is described in below.

First, genome of a test yeast is prepared. For this preparation, any known method such as Hereford method or potassium acetate method may be used (e.g., Methods in Yeast Genetics, Cold Spring Harbor Laboratory Press, 130 (1990)). Using a primer or a probe designed based on a nucleotide sequence (preferably, ORF sequence) of the gene encoding a protein having a vicinal diketone or diacetyl-reducing activity, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C. an annealing temperature of 4Q to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of producing total vicinal diketones or capability of producing total diacetyl of the yeast as determined by whether the molecular weight of the amplified product is a size that contains the DNA molecule of the specific part. In addition, by analyzing the nucleotide sequence of the amplified product, the capability may be predicted and/or assessed more precisely.

Moreover, in the present invention, a test yeast is cultured to measure an expression level of the gene having the nucleotide sequence of SEQ ID NO: 1 or SEQ ID NO: 3 and encoding a protein having a vicinal diketone or diacetyl-reducing activity to assess the test yeast for its capability of producing total vicinal diketones or capability of producing total diacetyl. In this case, the test yeast is cultured and then mRNA or a protein resulting from the gene encoding a protein having a vicinal diketone or diacetyl-reducing activity, is quantified. The quantification of mRNA or protein may be carried out by employing a known technique. For example, mRNA may be quantified, by Northern hybridization or quantitative RT-PCR, while protein may be quantified, for example, by Western blotting (Current Protocols in Molecular Biology, John Wiley & Sons 1994-2003).

Furthermore, test yeasts are cultured and expression levels of the gene of the present invention having the nucleotide sequence of SEQ ID NO: 1 or SEQ ID NO: 3 are measured to select a test yeast with the gene expression level according to the target capability of producing total vicinal diketones or capability of producing total diacetyl, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the gene having the nucleotide sequence of SEQ ID NO: 1 or SEQ ID NO: 3 and encoding a protein having a vicinal diketone or diacetyl-reducing activity, is measured in each yeast. By selecting a test yeast with the gene expressed higher than that in the reference yeast, a yeast suitable for brewing alcoholic beverages can be selected.

Alternatively, test yeasts are cultured and a yeast with a lower capability of producing total vicinal diketones or capability of producing total diacetyl, is selected, thereby selecting a yeast suitable for brewing desired alcoholic beverages.

In these cases, the test yeasts or the reference yeast may be, for example, a yeast introduced with the vector of the invention, a yeast with amplified expression of the gene of the present invention described above, a yeast with amplified expression of the protein of the present invention described above, an artificially mutated yeast or a naturally mutated yeast. The vicinal diketone or diacetyl-reducing activity can be assessed, for example by, a method of Drews, et al. (Drews et al., Mon. fur Brau., 34, 1966) wherein total VDK (DA and PD) concentration is measured in fermentation broth and compared. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine (see, e.g., Yasuji Oshima Ed., Biochemistry Experiments vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (e.g., *S*. *pastorianus, S. cerevisiae,* and *S. carlsbergensis*). *According* to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954 may be used. Further, whisky yeasts such as *Saccharomyces cerevisiae* NCYC90; wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning_ of Gene Encoding Protein Having Vicinal Diketone or Diacetyl-Reducing Activity (non-ScMMF1)

A specific novel gene encoding a vicinal diketone or diacetyl-reducing abilitiy (non-ScMMF1 gene; SEQ ID NO:1) from a lager brewing yeast was found, as a result of a search utilizing the comparison database described in Japanese Patent Application Laid-Open No. 2004-283169. Based on the acquired nucleotide sequence information, primers non-ScMMF1_F (SEQ ID NO: 5) and non-ScMMF1_R (SEQ ID NO: 6) were designed to amplify the full-length genes, respectively. PCR was carried out using chromosomal DNA of a genome sequencing strain, *Saccharomyces pastorianus* Weihenstephan 34/70 strain (hereinafter sometimes referred to as "W34/70 strain"), as a template to obtain DNA fragments including the full-length gene of non-ScMMF1.

The thus-obtained non-ScMMF1 gene fragment was inserted into pCR2.1-TOPO vector (manufactured by Invitrogen Corporation) by TA cloning. The nucleotide sequences of non-ScMMF1 gene were analyzed according to Sanger's method (F. Sanger, Science, 214: 1215, 1981) to confirm the nucleotide sequence.

### Example 2: Analysis of Expression of non-ScMMF1 Gene during Beer Fermentation

A beer fermentation test was conducted using a lager brewing yeast, *Saccharomyces pastorianus* 34/70 strain and then mRNA extracted from yeast cells during fermentation was analyzed by a yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

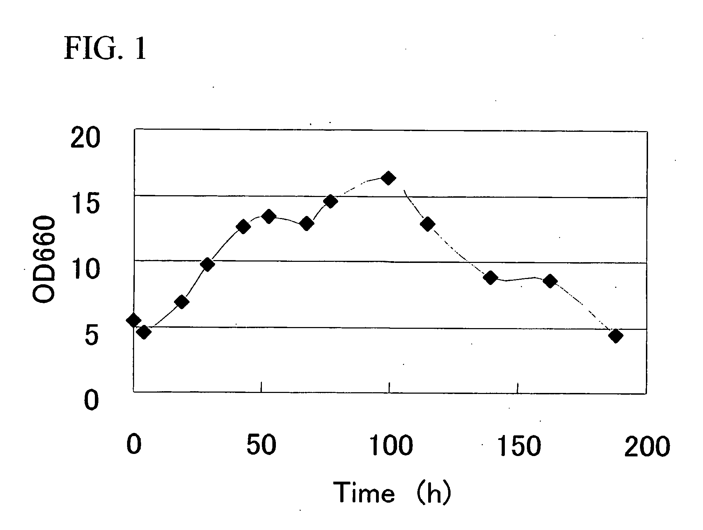

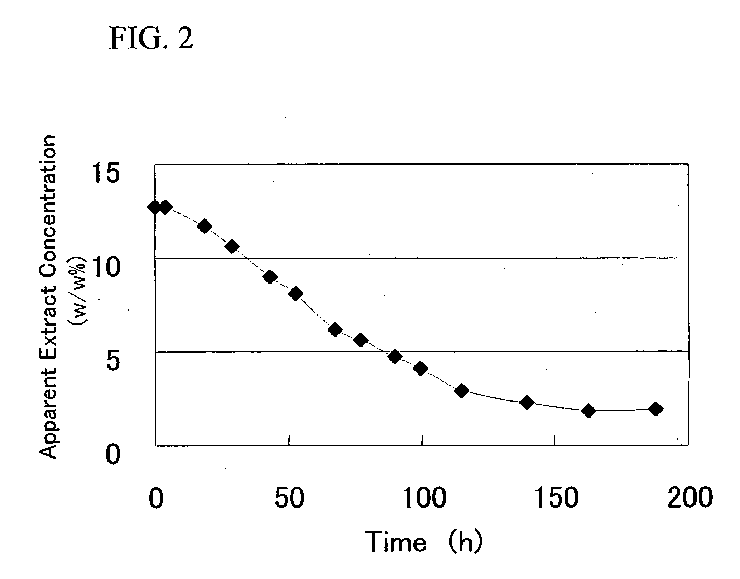

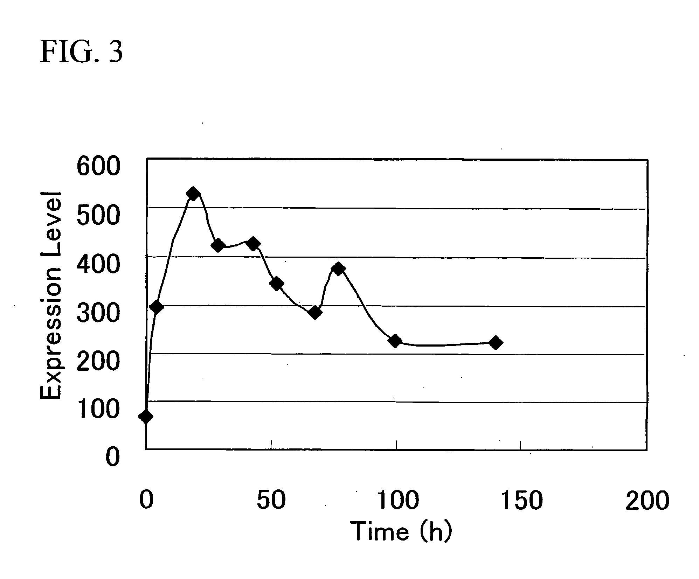

Sampling of fermentation liquid was performed with time, and variation with time of yeast growth amount (Fig. 1) and apparent extract concentration (Fig. 2) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of non-ScMMF1 gene is shown in Figure 3. As a result, it was confirmed that non-ScMMF1 gene was expressed in the general beer fermentation.

### Example 3: Preparation of non-ScMMF1 Gene-Highly Expressed Strain

The non-ScMMF1/pCR2.1-TOPO described in Example 1 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the non-ScMIMMMF1 high expression vector non-ScMMF1/pYCCPYNot. pYCGPYNot is the YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli.*

Using the non-ScMMF1 high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin, and designated as non-ScMMF1-highly expressed strain.

### Example 4: Analysis of Amount of VDKs Produced during Beer Fermentation

The parent strain and non-ScMMF1-highly expressed strain obtained in Example 3, are used to carry out fermentation test under the following conditions.

| | |
|---|---|
| Wort extract concentration | 11.85% |
| Wort content | 2 L |
| Wort dissolved oxygen concentration | 8 ppm |
| Fermentation temperature | 15°C, constant |
| Yeast pitching rate | 5 g wet yeast fungal body/L Wort |

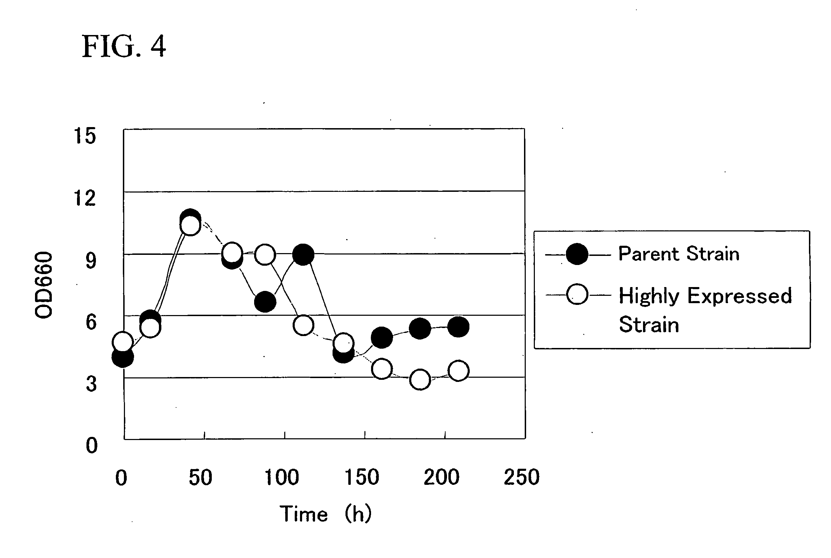

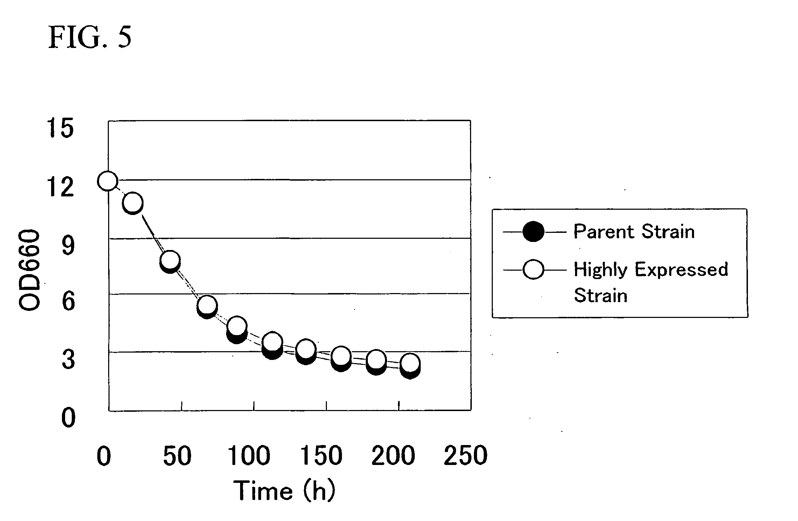

The fermentation broth was sampled with time to observe the cell growth (OD660) (Fig. 4) and extract consumption with time (Fig. 5). Quantification of the total VDKs in the fermentation broth was carried out by reacting VDKs (DA and PD) with hydroxylamine to produce glyoxime derivatives, then measuring absorbance of complexes formed from the reaction of resultant glyoxime derivatives and divalent ferric ions (Drews et al., Mon. fur Brau., 34, 1966). The precursors α-acetolactic-acid and α-acetohydroxybutyric-acid were previously converted by a gas washing method (oxidative decarboxylation method) to DA and PD, respectively, to quantify the total VDKs including them.

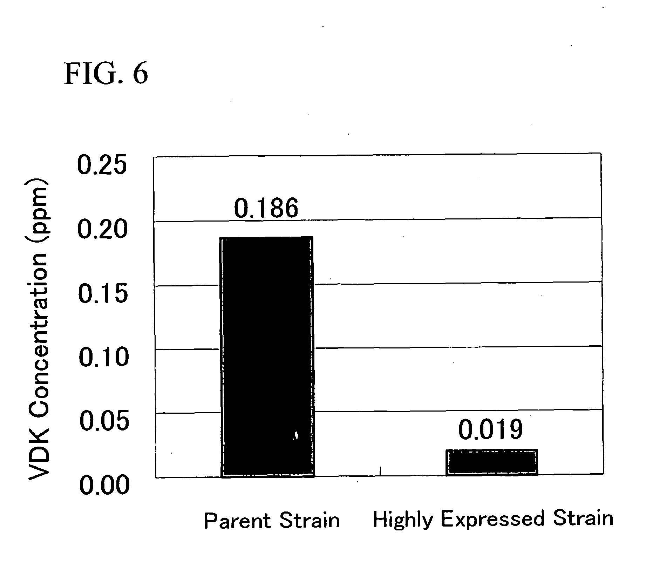

As shown in Fig. 6, use of the non-ScMMF 1-highly expressed strain reduced the total VDK concentration by about 90%, thereby the concentration being much lower than the threshold level in beer of 0.1ppm. In addition, significant differences were not observed between the parent strain and the highly expressed strain in cell growth and extract consumption in this testing.

### Example 5: Cloning of Gene Encoding Protein Having Vicinal Diketone or Diacetyl-Reducing Activity (ScMMF1)

A specific novel gene encoding a protein having a vicinal diketones or diacetyl-reducing ability (ScMMF1 gene; SEQ ID NO: 3) from a lager brewing yeast was found, as a result of a search utilizing the comparison database described in Japanese Patent Application Laid-Open No. 2004-283169. Based on the acquired nucleotide sequence information, primers ScMMF1_F (SEQ ID NO: 7) and ScMMF1_R (SEQ ID NO: 8) were designed to amplify the full-length genes, respectively. PCR was carried out using chromosomal DNA of a genome sequencing strain, *Saccharomyces pastorianus* Weihenstephan 34/70 strain, as a template to obtain DNA fragments including the full-length gene of ScMMF1.

The thus-obtained ScMMF1 gene fragment was inserted into pCR2.1-TOPO vector (manufactured by Invitrogen Corporation) by TA cloning. The nucleotide sequences of non-ScMMF1 gene were analyzed according to Sanger's method (F. Sanger, Science, 214: 1215, 1981) to confirm the nucleotide sequence.

### Example 6: Analysis of Expression of ScMMF1 Gene during Beer Fermentation

A beer fermentation test was conducted using a lager brewing yeast, *Saccharomyces pastorianus* 34/70 strain and then mRNA extracted from yeast cells during fermentation was analyzed by a yeast DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

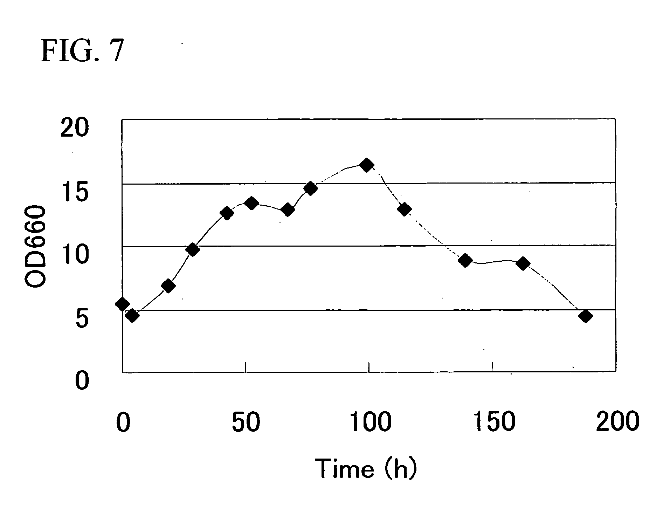

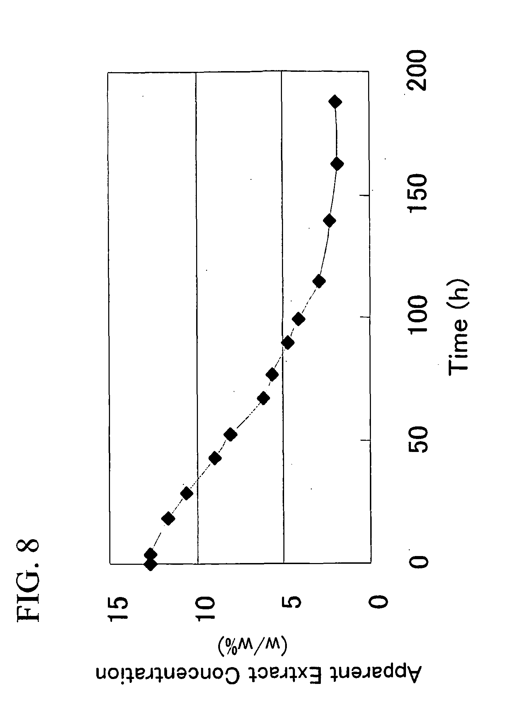

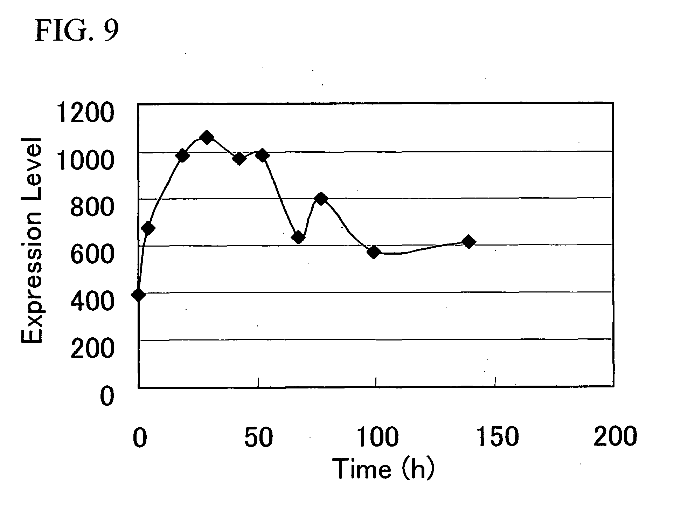

Sampling of fermentation liquid was performed with time, and variation with time of yeast growth amount (Fig. 7) and apparent extract concentration (Fig. 8) was observed. Simultaneously, yeast cells were sampled to prepare mRNA, and the prepared mRNA was labeled with biotin and was hybridized to a beer yeast DNA microarray. The signal was detected using GCOS; GeneChip Operating Software 1.0 (manufactured by Affymetrix Co.). Expression pattern of non-ScMMF1 gene is shown in Figure 9. As a result, it was confirmed that ScMMF1 gene was expressed in the general beer fermentation.

### Example 7: Preparation of ScMMF1-Highly Expressed Strain

The ScMMF1/pCR2.1-TOPO described in Example 5 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the ScMMF1 high expression vector non-ScMMF1/pYCGPYNot. pYCGPYNot is the YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli.*

Using the ScMMF1 high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 8: Analysis of Amount of VDks Produced during Beer Fermentation

The parent strain and ScMMF1-highly expressed strain obtained in Example 7, are used to carry out fermentation test under the following conditions.

| | |
|---|---|
| Wort extract concentration | 11.85% |
| Wort content | 2 L |
| Wort dissolved oxygen concentration | 8 ppm |
| Fermentation temperature | 15°C, constant |
| Yeast pitching rate | 5 g wet yeast fungal body/L Wort |

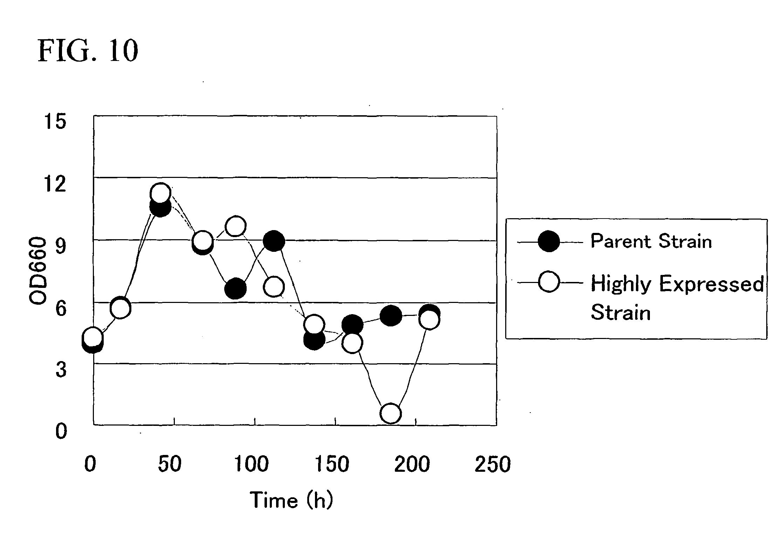

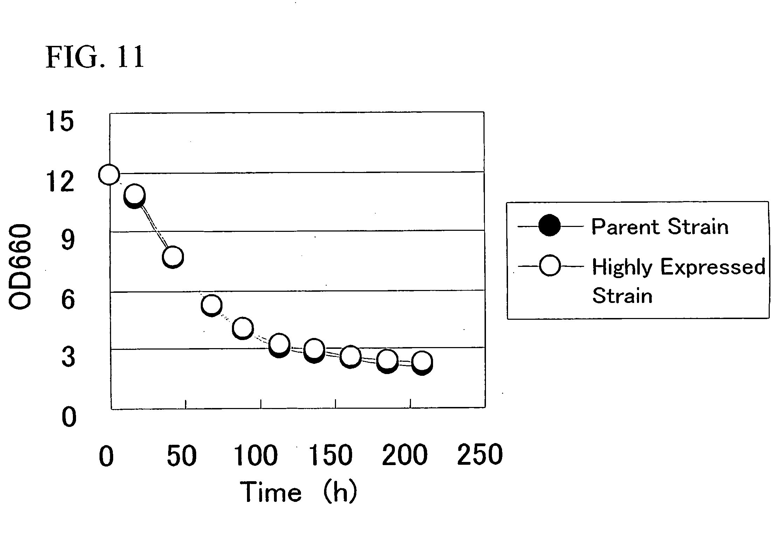

The fermentation broth was sampled with time to observe the cell growth (OD660) (Fig. 10) and extract consumption with time (Fig. 11). Quantification of the total VDKs in the fermentation broth was carried out by reacting VDKs (DA and PD) with hydroxylamine to produce glyoxime derivatives, then measuring absorbance of complexes formed from the reaction of resultant glyoxime derivatives and divalent ferric ions (Drews et al., Mon. fur Brau., 34, 1966). The precursors α-acetolactic-acid and α-acetohydroxybutyric-acid were previously converted by a gas washing method (oxidative decarboxylation method) to DA and PD, respectively, to quantify the total VDKs including them.

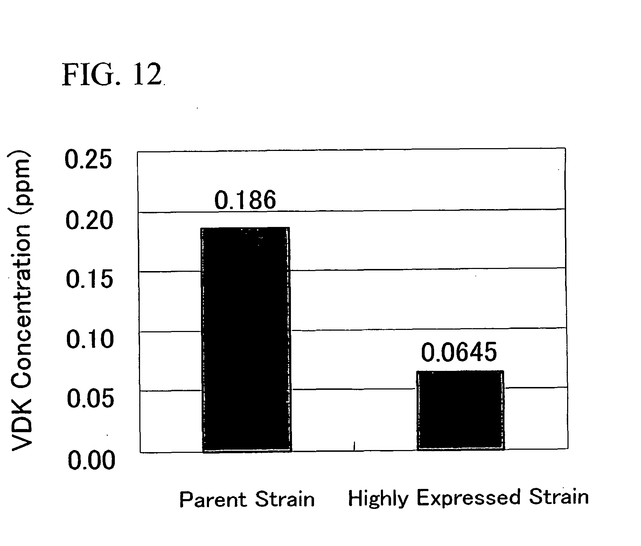

As shown in Fig. 12, use of the ScMMF1-highly expressed strain reduced the total VDK concentration by about 65%, thereby the concentration being much lower than the threshold level in beer of 0.1ppm. In addition, significant differences were not observed between the parent strain and the highly expressed strain in cell growth and extract consumption in this testing.

### INDUSTRIAL APPLICABILITY

According to the method for producing alcoholic beverages of the present invention, because of reduction of the production amount of VDKs, especially DA, which are responsible for off-flavors in products, alcoholic beverages with superior flavor can be readily produced.

## Claims

1. A polynucleotide selected from the group consisting of:
(a) a polynucleotide comprising a polynucleotide consisting of the nucleotide sequence of SEQ ID NO: 1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one to 8 amino acids thereof being deleted, substituted, inserted and/or added, and having an vicinal diketone or diacetyl-reducing activity; and
(d) a polynucleotide comprising a polynucleotide encoding a protein having an amino acid sequence having 94% or higher identity with the amino acid sequence of SEQ ID NO:2, and having a vicinal diketone or diacetyl-reducing activity.

2. The polynucleotide of Claim 1 selected from the group consisting of:
(g) a polynucleotide encoding a protein consisting of the amino acid sequence of SEQ ID NO: 2, or encoding an amino acid sequence of SEQ ID NO: 2 wherein 1 amino acid thereof is deleted, substituted, inserted, and/or added, and wherein said protein has a vicinal diketone or diacetyl-reducing activity; and
(h) a polynucleotide encoding a protein having 99% or higher identity with the amino acid sequence of SEQ ID NO: 2, and having a vicinal diketone or diacetyl-reducing activity.

3. The polynucleotide of Claim 1 comprising a polynucleotide consisting of SEQ ID NO: 1.

4. The polynucleotide of Claim 1 comprising a polynucleotide encoding a protein consisting of SEQ ID NO: 2.

5. The polynucleotide of any one of Claims 1 to 4, wherein the polynucleotide is DNA.

6. A protein encoded by the polynucleotide of any one of Claims 1 to 5.

7. A vector comprising the polynucleotide of any one of Claims 1 to 5.

8. A yeast comprising the vector of Claim 7.

9. The yeast of Claim 8, wherein a vicinal diketone or diacetyl-producing ability is reduced by introducing the vector of Claim 7.

10. The yeast of Claim 9, wherein a vicinal diketone or diacetyl-producing ability is reduced by increasing an expression level of the protein of Claim 6.

11. A method for producing an alcoholic beverage comprising culturing the yeast of any one of Claims 8 to 10.

12. A method for producing an alcoholic beverage with reduced amount of vicinal diketone or diacetyl, comprising culturing a yeast containing a vector comprising the polynucleotide selected from the group consisting of:
(j) a polynucleotide encoding a protein consisting of the amino acid sequence of SEQ ID NO: 4, or encoding an amino acid sequence of SEQ ID NO: 4 wherein 1 to 10 amino acids thereof is deleted, substituted, inserted, and/or added, and wherein said protein has a vicinal diketone or diacetyl-reducing activity;
(k) a polynucleotide encoding a protein having 90% or higher identity with the amino acid sequence of SEQ ID NO: 4, and having a vicinal diketone or diacetyl-reducing activity; and
(I) a polynucleotide which hybridizes to SEQ ID NO: 3 or which hybridizes to a nucleotide sequence complementary to the nucleotide sequence of SEQ ID NO: 3 under high stringent conditions, and which encodes a protein having a vicinal diketone or diacetyl-reducing activity.

13. The method for producing an alcoholic beverage of Claim 11 or 12, wherein the brewed alcoholic beverage is a malt beverage.

14. The method for producing an alcoholic beverage of Claim 11 or 12, wherein the brewed alcoholic beverage is wine.

15. A method for assessing a test yeast for its total vicinal diketone or total diacetyl-producing capability, comprising using a primer or a probe designed based on a nucleotide sequence of a gene having the nucleotide sequence of SEQ ID NO: 1 or SEQ ID NO: 3, and encoding a protein having a vicinal diketone or diacetyl-reducing ability.

16. A method for assessing a test yeast for its total vicinal diketone or total diacetyl-producing capability, comprising: culturing a test yeast; and measuring an expression level of a gene having the nucleotide sequence of SEQ ID NO: 1 or SEQ ID NO: 3, and encoding a protein having a vicinal diketone or diacetyl-reducing ability.

17. A method for selecting a yeast, comprising: culturing test yeasts; quantifying the protein according to Claim 6 or measuring an expression level of a gene having the nucleotide sequence of SEQ ID NO: 1 or SEQ ID NO: 3, and encoding a protein having a vicinal diketone or diacetyl-reducing ability; and selecting a test yeast having said protein amount or said gene expression level according to a target total vicinal diketone or total diacetyl-producing capability.

18. The method for selecting a yeast according to Claim 17, comprising: culturing a reference yeast and test yeasts; measuring an expression level of a gene having the nucleotide sequence of SEQ ID NO: 1 or SEQ ID NO: 3, and encoding a protein having a vicinal diketone or diacetyl-reducing ability in each yeast; and selecting a test yeast having the gene expressed higher than that in the reference yeast.

19. The method for selecting a yeast according to Claim 17, comprising: culturing a reference yeast and test yeasts; quantifying the protein according to Claim 6 in each yeast; and selecting a test yeast having said protein for a larger amount than that in the reference yeast.

20. A method for producing an alcoholic beverage comprising: conducting fermentation for producing an alcoholic beverage using the yeast according to any one of Claims 8 to 10 or a yeast selected by the method according to any one of Claims 17 to 19; and adjusting the total vicinal diketone or total diacetyl-producing capability.

## Patentansprüche

1. Polynucleotid ausgewählt aus der Gruppe bestehend aus:
(a) einem Polynucleotid umfassend ein Polynucleotid bestehend aus der Nucleotidsequenz von SEQ ID NO:1;
(b) einem Polynucleotid umfassend ein Polynucleotid, das ein Protein codiert, bestehend aus der Aminosäuresequenz von SEQ ID NO:2;
(c) einem Polynculeotid umfassend ein Polynucleotid, das ein Protein codiert, bestehend aus der Aminosäuresequenz von SEQ ID NO:2, wobei eine bis 8 Aminosäuren davon deletiert, substituiert, eingefügt und/oder hinzugefügt sind und das eine vicinales Diketon- oder Diacetyl-reduzierende Aktivität hat; und
(d) einem Polynucleotid umfassend ein Polynucleotid, das ein Protein codiert, das eine Aminosäuresequenz hat, die mit der Aminosäuresequenz von SEQ ID NO:2 eine Identität von 94% oder höher hat, und das eine vicinales Diketon- oder Diacetyl-reduzierende Aktivität hat.

2. Polynucleotid nach Anspruch 1 ausgewählt aus der Gruppe bestehend aus:
(g) einem Polynucleotid, das ein Protein codiert, das aus der Aminosäuresequenz von SEQ ID NO:2 besteht, oder eine Aminosäuresequenz von SEQ ID NO:2 codiert, in der eine Aminosäure davon deletiert, substituiert, eingefügt und/oder hinzugefügt ist, und wobei das Protein eine vicinales Diketon- oder Diacetyl-reduzierende Aktivität hat; und
(h) einem Polynucleotid, das ein Protein codiert, das mit der Aminosäuresequenz von SEQ ID NO:2 eine Identität von 99% oder höher hat, und das eine vicinales Diketon- oder Diacetyl-reduzierende Aktivität hat.

3. Polynucleotid nach Anspruch 1 umfassend ein Polynucleotid, das aus SEQ ID NO:1 besteht.

4. Polynucleotid nach Anspruch 1 umfassend ein Polynucleotid, das ein Protein codiert, das aus SEQ ID NO:2 besteht.

5. Polynucleotid nach einem der Ansprüche 1 bis 4, wobei das Polynucleotid DNA ist.

6. Protein codiert durch das Polynucleotid nach einem der Ansprüche 1 bis 5.

7. Vektor umfassend das Polynucleotid nach einem der Ansprüche 1 bis 5.

8. Hefe umfassend den Vektor nach Anspruch 7.

9. Hefe nach Anspruch 8, wobei eine vicinales Diketon- oder Diacetyl-herstellende Fähigkeit durch Einbringen des Vektors nach Anspruch 7 reduziert ist.

10. Hefe nach Anspruch 9, wobei eine vicinales Diketon- oder Diacetyl-herstellende Fähigkeit durch Erhöhung eines Expressionsspiegels des Proteins nach Anspruch 6 reduziert ist.

11. Verfahren zur Herstellung eines alkoholischen Getränks umfassend das Züchten der Hefe nach einem der Ansprüche 8 bis 10.

12. Verfahren zur Herstellung eines alkoholischen Getränks mit reduzierter Menge an vicinalem Diketon oder Diacetyl, umfassend das Züchten einer Hefe, die einen Vektor enthält, der das Polynucleotid umfasst, das ausgewählt ist aus der Gruppe bestehend aus:
(j) einem Polynucleotid, das ein Protein codiert, das aus der Aminosäuresequenz von SEQ ID NO:4 besteht, oder eine Aminosäuresequenz von SEQ ID NO:4 codiert, in der 1 bis 10 Aminosäuren davon deletiert, substituiert, eingefügt und/oder hinzugefügt ist, und wobei das Protein eine vicinales Diketon- oder Diacetyl-reduzierende Aktivität hat;
(k) einem Polynucleotid, das ein Protein codiert, das mit der Aminosäuresequenz von SEQ ID NO:4 eine Identität von 90% oder höher hat, und das eine vicinales Diketon- oder Diacetyl-reduzierende Aktivität hat; und
(l) einem Polynucleotid, das mit SEQ ID NO:3 hybridisiert oder das unter hochstringenten Bedingungen mit einer Nucleotidsequenz hybridisiert, die komplementär ist zu der Nucleotidsequenz von SEQ ID NO:3, und das ein Protein codiert, das eine vicinales Diketon- oder Diacetyl-reduzierende Aktivität hat.

13. Verfahren zur Herstellung eines alkoholischen Getränks nach Anspruch 11 oder 12, wobei das gebraute alkoholische Getränk ein Malzgetränk ist.

14. Verfahren zur Herstellung eines alkoholischen Getränks nach Anspruch 11 oder 12, wobei das gebraute alkoholische Getränk Wein ist.

15. Verfahren zur Bewertung einer Testhefe bezüglich ihrer vollständigen vicinales Diketon- oder Diacetyl-herstellenden Fähigkeit, umfassend die Verwendung eines Primers oder einer Sonde, der/die konzipiert ist basierend auf einer Nucleotidsequenz eines Gens, das die Nucleotidsequenz von SEQ ID NO:1 oder SEQ ID NO:3 hat und ein Protein codiert, das eine vicinales Diketon- oder Diacetyl-reduzierende Fähigkeit hat.

16. Verfahren zur Bewertung einer Testhefe bezüglich ihrer vollständigen vicinales Diketon- oder Diacetyl-herstellender Fähigkeit, umfassend: das Züchten einer Testhefe; und das Messen eines Expressionsspiegels eines Gens, das die Nucleotidsequenz von SEQ ID NO:1 oder SEQ ID NO:3 hat und ein Protein codiert, das eine vicinales Diketon- oder Diacetyl-reduzierende Fähigkeit hat.

17. Verfahren zur Auswahl eine Hefe umfassend: das Züchten von Testhefen; das Quantifizieren des Proteins nach Anspruch 6 oder das Messen eines Expressionsspiegels eines Gens, das die Nucleotidsequenz von SEQ ID NO:1 oder SEQ ID NO:3 hat und ein Protein codiert, das eine vicinales Diketon- oder Diacetyl-reduzierende Fähigkeit hat; und das Auswählen einer Testhefe, die die Proteinmenge oder den Genexpressionsspiegel gemäß einer angestrebten vollständigen vicinales Diketon- oder Diacetyl-herstellenden Fähigkeit hat.

18. Verfahren zur Auswahl einer Hefe nach Anspruch 17, umfassend: das Züchten einer Referenzhefe und von Testhefen; das Messen in jeder Hefe eines Expressionsspiegels eines Gens, das die Nucleotidsequenz von SEQ ID NO:1 oder SEQ ID NO:3 hat und ein Protein codiert, das eine vicinales Diketon- oder Diacetyl-reduzierende Fähigkeit hat; und das Auswählen einer Testhefe, die das Gen höher exprimiert hat als das in der Referenzhefe.

19. Verfahren zur Auswahl einer Hefe nach Anspruch 17, umfassend: das Züchten einer Referenzhefe und von Testhefen; das Quantifizieren des Proteins nach Anspruch 6 in jeder Hefe; und das Auswählen einer Testhefe, die das Protein in einer größeren Menge hat als das in der Referenzhefe.

20. Verfahren zur Herstellung eines alkoholischen Getränks, umfassend: das Durchführen einer Fermentation zur Herstellung eines alkoholischen Getränks unter Verwendung der Hefe nach einem der Ansprüche 8 bis 10 oder einer Hefe, die ausgewählt ist durch das Verfahren nach einem der Ansprüche 17 bis 19; und das Einstellen der vollständigen vicinales Diketon- oder Diacetyl-herstellenden Fähigkeit.

## Revendications

1. Polynucléotide choisi dans le groupe constitué par :
(a) un polynucléotide comprenant un polynucléotide constitué de la séquence nucléotidique de SEQ ID NO : 1 ;
(b) un polynucléotide comprenant un polynucléotide codant pour une protéine constituée de la séquence d'acides aminés de SEQ ID NO : 2 ;
(c) un polynucléotide comprenant un polynucléotide codant pour une protéine constituée de la séquence d'acides aminés de SEQ ID NO : 2 avec un à 8 acides aminés de celle-ci étant supprimés, substitués, insérés et/ou ajoutés, et possédant une activité de réduction de dicétone vicinale ou de diacétyle ; et
(d) un polynucléotide comprenant un polynucléotide codant pour une protéine ayant une séquence d'acides aminés ayant 94 % ou plus d'identité avec la séquence d'acides aminés de SEQ ID NO : 2, et possédant une activité de réduction de dicétone vicinale ou de diacétyle.

2. Polynucléotide selon la revendication 1 choisi dans le groupe constitué par :
(g) un polynucléotide codant pour une protéine constituée de la séquence d'acides aminés de SEQ ID NO : 2, ou codant pour une séquence d'acides aminés de SEQ ID NO : 2 dans laquelle 1 acide aminé de celle-ci est supprimé, substitué, inséré et/ou ajouté, et où ladite protéine possède une activité de réduction de dicétone vicinale ou de diacétyle ; et
(h) un polynucléotide codant pour une protéine ayant 99 % ou plus d'identité avec la séquence d'acides aminés de SEQ ID NO : 2, et possédant une activité de réduction de dicétone vicinale ou de diacétyle.

3. Polynucléotide selon la revendication 1 comprenant un polynucléotide constitué de SEQ ID NO : 1.

4. Polynucléotide selon la revendication 1 comprenant un polynucléotide codant pour une protéine constituée de SEQ ID NO : 2.

5. Polynucléotide selon l'une quelconque des revendications 1 à 4, où le polynucléotide est de l'ADN.

6. Protéine codée par le polynucléotide selon l'une quelconque des revendications 1 à 5.

7. Vecteur comprenant le polynucléotide selon l'une quelconque des revendications 1 à 5.

8. Levure comprenant le vecteur selon la revendication 7.

9. Levure selon la revendication 8, dans laquelle une capacité de production de dicétone vicinale ou de diacétyle est réduite par l'introduction du vecteur selon la revendication 7.

10. Levure selon la revendication 9, dans laquelle une capacité de production de dicétone vicinale ou de diacétyle est réduite en augmentant un niveau d'expression de la protéine selon la revendication 6.

11. Procédé de production d'une boisson alcoolisée comprenant la culture de la levure selon l'une quelconque des revendications 8 à 10.

12. Procédé de production d'une boisson alcoolisée présentant une quantité réduite de dicétone vicinale ou de diacétyle, comprenant la culture d'une levure contenant un vecteur comprenant le polynucléotide choisi dans le groupe constitué par :
(j) un polynucléotide codant pour une protéine constituée de la séquence d'acides aminés de SEQ ID NO : 4, ou codant pour une séquence d'acides aminés de SEQ ID NO : 4 dans laquelle 1 à 10 acides aminés de celle-ci est supprimé, substitué, inséré et/ou ajouté, et où ladite protéine possède une activité de réduction de dicétone vicinale ou de diacétyle ;
(k) un polynucléotide codant pour une protéine ayant 90 % ou plus d'identité avec la séquence d'acides aminés de SEQ ID NO : 4, et possédant une activité de réduction de dicétone vicinale ou de diacétyle ; et
(l) un polynucléotide qui s'hybride à SEQ ID NO : 3 ou qui s'hybride à une séquence nucléotidique complémentaire de la séquence nucléotidique de SEQ ID NO : 3 dans des conditions stringentes élevées, et qui code pour une protéine possédant une activité de réduction de dicétone vicinale ou de diacétyle.

13. Procédé de production d'une boisson alcoolisée selon la revendication 11 ou 12, dans lequel la boisson alcoolisée brassée est une boisson à base de malt.

14. Procédé de production d'une boisson alcoolisée selon la revendication 11 ou 12, dans lequel la boisson alcoolisée brassée est du vin.

15. Procédé d'évaluation d'une levure à tester pour sa capacité de production de dicétone vicinale totale ou de diacétyle total, comprenant l'utilisation d'une amorce ou d'une sonde conçue sur la base d'une séquence nucléotidique d'un gène ayant la séquence nucléotidique de SEQ ID NO : 1 ou SEQ ID NO : 3, et codant pour une protéine possédant une capacité de réduction de dicétone vicinale ou de diacétyle.

16. Procédé d'évaluation d'une levure à tester pour sa capacité de production de dicétone vicinale totale ou de diacétyle total, comprenant : la culture d'une levure à tester ; et la mesure d'un taux d'expression d'un gène ayant la séquence nucléotidique de SEQ ID NO : 1 ou SEQ ID NO : 3, et codant pour une protéine possédant une capacité de réduction de dicétone vicinale ou de diacétyle.

17. Procédé de sélection d'une levure, comprenant : la culture des levures à tester ; la quantification de la protéine selon la revendication 6 ou la mesure d'un taux d'expression d'un gène ayant la séquence nucléotidique de SEQ ID NO : 1 ou SEQ ID NO : 3, et codant pour une protéine possédant une capacité de réduction de dicétone vicinale ou de diacétyle ; et la sélection d'une levure à tester ayant ladite quantité de protéine ou ledit taux d'expression de gène selon une capacité cible de production de dicétone vicinale totale ou de diacétyle total.

18. Procédé de sélection d'une levure selon la revendication 17, comprenant : la culture d'une levure de référence et des levures à tester ; la mesure d'un taux d'expression d'un gène ayant la séquence nucléotidique de SEQ ID NO : 1 ou SEQ ID NO : 3, et codant pour une protéine possédant une capacité de réduction de dicétone vicinale ou de diacétyle dans chaque levure ; et la sélection d'une levure à tester ayant le gène exprimé de manière plus élevée que dans la levure de référence.

19. Procédé de sélection d'une levure selon la revendication 17, comprenant : la culture d'une levure de référence et des levures à tester ; la quantification de la protéine selon la revendication 6 dans chaque levure ; et la sélection d'une levure à tester ayant ladite protéine en une quantité supérieure à celle dans la levure de référence.

20. Procédé de production d'une boisson alcoolisée comprenant : la mise en oeuvre d'une fermentation pour la production d'une boisson alcoolisée en utilisant la levure selon l'une quelconque des revendications 8 à 10 ou une levure sélectionnée par le procédé selon l'une quelconque des revendications 17 à 19 ; et l'ajustement de la capacité de production de dicétone vicinale totale ou de diacétyle total.


### SEQUENCE LISTING

<110> Suntory Limited
<120> Gene encoding protein with vicinal diketone or diacetyl-reducing activity and use thereof
<130> PCT06-0128
<150> JP2006-48957
   <151>
<160> 8
<170> PatentIn version 3.3
<210> 1
   <211> 438
   <212> DNA
   <213> Saccharomyces sp.
<400> 1
   atgtttctaa gaaattccgt tttgagaacc accccaatct tgaaaagggg tttgacaaca      60
   ttgaccccgg tcagcaccaa actggcacca cctgctgccg cctcttattc ccaggccatg     120
   aaggcgaaca acttcgtcta cgtgtctggt caaatcccat ataccgcaga aaacaagcct     180
   gttcaaggtt ctatctctga caaggcggaa caagttttcc aaaacgtcaa gaacatcttg     240
   gctgaaagca actcgtcttt gaacagcgtt gtcaaagtca acgttttctt agctgatatg     300
   aaaaactttg ctgaattcaa ttcagtgtat gccaaacact tccacaccca caagcctgca     360
   agatcatgcg tcggtgttgc ttctttgcca ttgaacgttg atttggaaat ggaagtcatt     420
   gctgttgaaa agaattga                                                   438
<210> 2
   <211> 145
   <212> PRT
   <213> Saccharomyces sp.
<400> 2
   Met Phe Leu Arg Asn Ser Val Leu Arg Thr Thr Pro Ile Leu Lys Arg
   1               5                   10                  15
   Gly Leu Thr Thr Leu Thr Pro Val Ser Thr Lys Leu Ala Pro Pro Ala
               20                  25                  30
   Ala Ala Ser Tyr Ser Gln Ala Met Lys Ala Asn Asn Phe Val Tyr Val
           35                  40                  45
   Ser Gly Gln Ile Pro Tyr Thr Ala Glu Asn Lys Pro Val Gln Gly Ser
       50                  55                  60
   Ile Ser Asp Lys Ala Glu Gln Val Phe Gln Asn Val Lys Asn Ile Leu
   65                  70                  75                  80
   Ala Glu Ser Asn Ser Ser Leu Asn Ser Val Val Lys Val Asn Val Phe
                   85                  90                  95
   Leu Ala Asp Met Lys Asn Phe Ala Glu Phe Asn Ser Val Tyr Ala Lys
               100                 105                 110
   His Phe His Thr His Lys Pro Ala Arg Ser Cys Val Gly Val Ala Ser
           115                 120                 125
   Leu Pro Leu Asn Val Asp Leu Glu Met Glu Val Ile Ala Val Glu Lys
       130                 135                 140
   Asn
   145
<210> 3
   <211> 438
   <212> DNA
   <213> Saccharomyces sp.
<400> 3
   atgtttttaa gaaattccgt tttgagaaca gcaccagtct tgaggagggg tataacaaca 60
   ttgactccgg tcagcaccaa gttggcccca cccgctgccg cctcttactc ccaagctatg 120
   aaggccaaca attttgtgta cgtgtctggt caaatccctt atactccaga taacaagcct 180
   gttcaaggtt ctatctctga gaaggccgaa caagtttttc aaaacgttaa gaatatctta 240
   gcagaaagta attcttcttt agacaatata gtcaaagtca acgtattctt ggctgacatg 300
   aaaaactttg ccgaattcaa ctctgtatac gccaagcact tccacaccca taagcctgca 360
   agatcctgtg ttggtgttgc ttccttacct ttgaatgttg atttagaaat ggaagttatc 420
   gctgttgaaa agaattga 438
<210> 4
   <211> 145
   <212> PRT
   <213> Saccharomyces sp.
<400> 4
   Met Phe Leu Arg Asn Ser Val Leu Arg Thr Ala Pro Val Leu Arg Arg
   1               5                   10                  15
   Gly Ile Thr Thr Leu Thr Pro Val Ser Thr Lys Leu Ala Pro Pro Ala
               20                  25                  30
   Ala Ala Ser Tyr Ser Gln Ala Met Lys Ala Asn Asn Phe Val Tyr Val
           35                  40                  45
   Ser Gly Gln Ile Pro Tyr Thr Pro Asp Asn Lys Pro Val Gln Gly Ser
       50                  55                  60
   Ile Ser Glu Lys Ala Glu Gln Val Phe Gln Asn Val Lys Asn Ile Leu
   65                  70                  75                  80
   Ala Glu Ser Asn Ser Ser Leu Asp Asn Ile Val Lys Val Asn Val Phe
                   85                  90                  95
   Leu Ala Asp Met Lys Asn Phe Ala Glu Phe Asn Ser Val Tyr Ala Lys
               100                 105                 110
   His Phe His Thr His Lys Pro Ala Arg Ser Cys Val Gly Val Ala Ser
           115                 120                 125
   Leu Pro Leu Asn Val Asp Leu Glu Met Glu Val Ile Ala Val Glu Lys
       130                 135                 140
   Asn
   145
<210> 5
   <211> 40
   <212> DNA
   <213> Artificial
<220>
   <223> Primer
<400> 5
   gagctcatag cggccatgtt tctaagaaat tccgttttga 40
<210> 6
   <211> 42
   <212> DNA
   <213> Artificial
<220>
   <223> Primer
<400> 6
   ggatcctatg cggccgctgt tttatatacg ctaaagattt gc 42
<210> 7
   <211> 40
   <212> DNA
   <213> Artificial
<220>
   <223> Primer
<400> 7
   gagctcatag cggccatgtt tttaagaaat tccgttttga 40
<210> 8
   <211> 42
   <212> DNA
   <213> Artificial
<220>
   <223> Primer
<400> 8
   ggatcctatg cggccgctaa aatcttatat acgctaaaga at 42